# EUROPEAN PATENT SPECIFICATION

(11) **EP 4 180 575 B1**
(45) Date of publication and mention of the grant of the patent: **09.07.2025**
(21) Application number: 21838828.8
(22) Date of filing: 09.07.2021
(51) Int. Cl.: G01N 33/28, G01N 31/22

(54) **AVIATION FUEL FREE WATER TEST PAPER, PREPARATION METHOD THEREFOR AND DETECTION DEVICE THEREOF**
TESTPAPIER FÜR KRAFTSTOFFFREIES WASSER FÜR DIE LUFTFAHRT, HERSTELLUNGSVERFAHREN DAFÜR UND DETEKTIONSVORRICHTUNG DAFÜR
PAPIER RÉACTIF D'EAU LIBRE DE CARBURANT POUR L'AVIATION, SON PROCÉDÉ DE PRÉPARATION ET DISPOSITIF DE DÉTECTION ASSOCIÉ

(30) Priority: 10.07.2020 CN 202010664021
(43) Date of publication of application: 17.05.2023
(73) Proprietor: The Second Research Institute of CAAC, Chengdu, Sichuan 610041 (CN)
(72) Inventor: LIU, Hua, Chengdu, Sichuan 610225 (CN); XIA, Zuxi, Chengdu, Sichuan 610225 (CN); WANG, Wenwu, Chengdu, Sichuan 610225 (CN); XIANG, Hai, Chengdu, Sichuan 610225 (CN); ZHOU, Ning, Chengdu, Sichuan 610225 (CN); ZENG, Tai, Chengdu, Sichuan 610225 (CN)
(74) Representative: Chung, Hoi Kan
(86) International application number: PCT/CN2021/105477
(87) International publication number: WO 2022/007948

(56) References cited:
- CN-A- 1 113 981
- CN-A- 106 990 105
- CN-A- 111 077 036
- CN-A- 111 851 144
- CN-Y- 201 392 317
- RU-C1- 2 375 713
- US-A- 2 844 025

## Description

### FIELD OF THE INVENTION

The present invention relates to the technical field of aerospace, in particular to preparing a test paper for detecting free water in aviation fuels and a detection device.

### BACKGROUND OF THE INVENTION

Aviation fuel, including aviation gasoline and aviation kerosene, often contains dissolved water and free water. The free water is also referred to as non-dissolved water. The free water and the dissolved water in the fuel are converted into each other in the fuel with the change of temperature and humidity in such manners that: the free water is dissolved in the fuel and converted into the dissolved water as the temperature of the fuel increases, further increasing content of the dissolved water, and the dissolved water is separated from the fuel and converted into free water as the temperature of the fuel decreases. The dissolved water does not change the appearance of the fuel, whereas the free water is generally shown as vaporous or turbid form, water droplets, water masses (layers), and visually invisible superfine water droplets dispersed and suspended in the fuel. The dissolved water and the free water in the fuel can be changed at various stages of transportation, storage and use process of the fuel owing to changes in atmosphere temperature and humidity, for example, owing to residual water after cleaning of transportation devices (such as oil pipeline, oil tank truck, oil tanker, etc.) and oil storage tank or influence by rain, snow or high-humidity atmosphere due to poor sealing of devices, the content of water in the oil product increases. The dissolved water in the fuel does not directly affect the use safety of the fuel. However, the free water has a significant impact on operation safety of the aeronautical power system, and may even, in severe case, lead to a fatal accident of catastrophic crash.

The hazards of the free water in aviation fuel are mainly manifested in aspects as follows.
(1) The oxidation rate of the aviation fuel is accelerated, and especially in presence of metal particles such as iron and copper, the presence of water accelerates the oxidation of aviation fuel, producing viscous compounds (commonly referred to as oil sludge) to affect its physical and chemical properties.
(2) Microorganisms can be bred in water, causing further contamination of aviation fuel. For example, in 2011, microbial contamination of fuel at Ben-Gurion airport in Israel prevented all aircraft on the route from refueling in Israel.
(3) Water can freeze at low temperature, and freezing may cause clogging of the oil pipeline, thereby causing high-altitude failure of engine. For example, in January 2008, the fuel pipelines of a Boeing 777 aircraft at Heathrow Airport in the United Kingdom were frozen and clogged during high-altitude flight due to water-containing aviation fuel, resulting in stalling landing of the aircraft outside the runway and causing serious damage to the aircraft and injury to personnel.
(4) The free water in the fuel can reduce the viscosity of the aviation fuel and affect the effect of antistatic additive. During high-speed transportation, the aviation fuel may rub against non-metallic pipeline and generate and accumulate free charges, which may bring explosion.
(5) Corrosive gases such as SO₂ in atmosphere are easily dissolved in water to corrode electrical components.

Have described above reasons, dewatering of aviation fuel and detection of free water in the aviation fuel are required throughout the whole process of production, storage and transportation and refueling of the aircraft. According to the rules and standards of the international civil aviation industry, when fuel is added to an aircraft fuel tank, moisture testing must be carried out to strictly control the content of the free water so as to ensure cleanliness and dryness of the fuel product. According to stipulation of International Air Transport Association (IATA), the content of the free water in the fuel should be lower than 30 ppm. According to stipulation of American Aeronautical Association (A4A), the content of the free water in the fuel should be lower than 15 ppm. According to stipulations of International Joint Inspection Group (JIG) and China Civil Aviation Standard, the test paper of a chemical water meter does not change in color when the content of the free water in the fuel is detected by using the chemical water meter, meaning that the content of the free water in the fuel is lower than 10 ppm. Therefore, it is particularly important to prepare a water meter with simple and convenient operation and fast result acquisition.

Currently, there are two types of methods of aviation fuel moisture detectors recommended by IATA. The first type is to use direct color development of pigment, such as dye crystal violet, lucifer yellow, auramine 0.150, rhodamine B500 and the like, in water. The second type is to use the color change of the compound when it is dissolved in water to reflect present free water and its content. For example, anhydrous copper sulfate is white compound, and forms copper ion-water chelate upon contact with water to become blue. There were aviation fuel moisture detectors developed by Shell International Trading Co., Ltd. (U.K.), EXXON. (U.S.A.), Scott Manufacturing Co., Ltd. and Gammon Technical Products Inc., but these moisture detectors have limitations of high price, short effective period, overelaborate auxiliary components, complex structure, specialized operation requirement and inconvenient use in flight zone. In addition, a water measuring method is described in US3.066.211, in which, a fluorescent substance is adopted to be dissolved in free water in fuel, ultraviolet lamp irradiation is carried out to measure fluorescence intensity, and the content of the free water is measured according to the relationship between the fluorescence intensity and the content of the free water. The similar method is used by an AQUA-GLO water meter of the GTP Company, America. The method requires the use of proprietary equipment for detection. The detection process is complex and time-consuming. The process is not suitable for rapid detection at aircraft refueling site. The water meter of Shell, UK adopts water test paper, which is prepared by coating a water-sensitive reagent on filter paper and utilizes negative pressure to make fuel flow through the water-sensitive test paper for free water content test. When the water content is lower than 10 ppm, there is no color change; when the water content is higher than 15 ppm, there is no significant color change on the test paper; and when the water content is 30 ppm, there is a relatively obvious color change. Since the water-sensitive reagent on the surface of the test paper of the Shell water meter is easily peeled off and oxidized, its storage effective period is short, and it is difficult to judge failure of the test paper, and there is a risk in application. In summary, the conventional detection method has problems of complex detection, high cost, long detection period and short storage period.

RU2375713 discloses applying a mixture of K₃[Fe(CN)₆] and FeSO₄ to a test element, for detecting water in fuel.

### OBJECTS AND SUMMARY OF THE INVENTION

Therefore, the present invention provides a method for preparing a test paper to detect free water in aviation fuels, comprising the steps of: soaking a filter paper in an aqueous solution of K₃[Fe(CN)₆]; drying the filter paper to obtain an intermediate test paper; and coating a surface of the intermediate test paper with a powdery mixture of FeSO₄ and a chelator, wherein a weight ratio of the K₃[Fe(CN)₆] to the powdery mixture of the FeSO₄ and the chelator is 1 : (5-50).

Further, a concentration of the aqueous solution of K₃[Fe(CN)₆] solution is 3 - 10 g/L.

Further, in the drying step, drying the filter paper at 40 - 80°C for 20 - 60 minutes to obtain the intermediate test paper.

Further, the method comprises preparing the powdery mixture of the FeSO₄ and the chelator, including the steps of: mixing the FeSO₄ and the chelator; and milling the powdery mixture of the FeSO₄ and the chelator until the powdery mixture has a particle size of 20 - 50 µm.

The present invention also provides a device for detecting free water in aviation fuels. The device comprises the test paper prepared with the method described above. The device further comprises a housing, a core sleeve being inserted into the housing to form a sealing connection, a cavity formed between a front surface of the housing and a front surface of the core sleeve. The device further comprises a suction hole which passes through the core sleeve. The device further comprises a fuel inlet which passes through the front surface of the housing. The cavity communicates with the suction hole and with the fuel inlet. The test paper is configured in the cavity. The aviation fuel being tested passes by the test paper along a fuel channel formed by the fuel inlet, the cavity and the suction hole.

Further, a diameter of the fuel inlet is 3 - 7 mm.

Compared with the prior art, the test paper for detecting free water in aviation fuels and preparation method thereof provided by the present invention have the beneficial effects as follows.
(1) The detection method is simple (only by suction-filtering to-be-detected aviation fuels, passing by the test paper and observing color change of the test paper), and has simple and easy operation, no need of specialization, no need of instrument, convenient use, naked eye intuitive and concise detection, fast detection and high efficiency.
(2) The test paper has long storage time and long effective service life (up to 15 months), is easy to be judged whether failure or not through intuitive color change. Detection reliability is improved.
(3) The test paper, which has light yellow base color, produces more obvious color change when in contact with water for easy judgment.
(4) The test paper has high detection sensitivity, can realize the color change process (when in contact with water) as yellow (0 ppm), few light yellowish-green spots (5 ppm), light yellowish-green spots (10 ppm), yellowish-green spots (20 ppm), green spots (30 ppm) and blue spots (40 ppm), is green when the content of free water is 30 ppm (critical value) and is blue when the content of free water is 40 ppm, has color development at low free water content (lower than 20 ppm, such as 10 ppm), and has obvious color change, fast and sensitive color development and easy judgment. At the critical value, the green is stable and is maintained for more than 24 hours under dry condition.
(5) Raw materials are easily available, and the product has low cost.

### BRIEF DESCRIPTION OF FIGURES

In order to illustrate the technical solutions of the embodiments of the present invention more clearly, a brief introduction to the figures used in the embodiments is given below. Notably, the figures in the following description are merely illustrative of some embodiments of the present invention, and other figures can be made to those of ordinary skill in the art without involving any inventive effort.
Fig. 1 schematic sectional view of the device for detecting free water in aviation fuels according to an embodiment of the invention;
Fig. 2 is detection results of aviation fuels with different free water contents detected by the test paper prepared in Example 1 of the present invention;
Fig. 3 is a picture of the test paper prepared in Comparative Example 4 of the present invention;
Fig. 4 is Fig.4 is a picture of the test paper prepared in Comparative Example 5 of the present invention;
Fig. 5 is Fig.5 is a scanning electron microscope photograph of the test paper prepared in Example 1 of the present invention;
Fig.6 is a scanning electron microscope photograph of the test paper prepared in Comparative Example 11 of the present invention; and
Fig.7 is a scanning electron microscope photograph of the test paper prepared in Comparative Example 12 of the present invention.

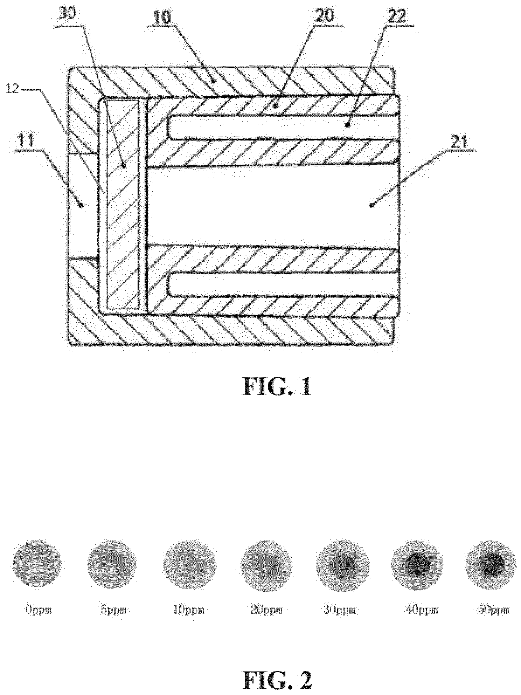

Description of the figure reference signs in FIG.1: 10) housing; 11) fuel inlet; 12) cavity; 20) core sleeve; 21) suction hole; 22) annular inner groove; and 30) test paper.

### DETAILED DESCRIPTION OF THE EMBODIMENTS

Embodiments of the present invention are described in detail with reference to the figures.

It should be noted that the following embodiments and the characteristics in the embodiments can be combined with each other without conflict. Based on the embodiments in the disclosure, all other embodiments obtained by those of ordinary skill in the art without involving any inventive effort fall within the protection scope of the disclosure.

The method of the present invention provides a test paper for detecting free water in aviation fuels, comprising a filter paper, and K₃[Fe(CN)₆] and powdery mixture of FeSO₄ and a chelator, which are loaded on the surface of the filter paper.

The color development principle of the test paper for testing free water in aviation fuels provided by the present invention is shown as follows.

K₃[Fe(CN)₆] and FeSO₄ chemically react in an aqueous solution to form indigo. The chemical equation is shown as

2[Fe(CN)₆]³⁻+3Fe²⁺=Fe₃[Fe(CN)₆]₂ (I)

Especially, K₃[Fe(CN)₆] and FeSO₄ do not react in solid state. When water is present, K₃[Fe(CN)₆] and FeSO₄ are electrolyzed to form [Fe(CN)]³⁻ and Fe²⁺, respectively, and then react rapidly to form indigo. There is a corresponding relationship between the amount of indigo produced by the chemical reaction and water content: when the water content is high, the dissolved masses of the two substances are large, the mass of the formed indigo is increased, and the color change is more obvious. Based on the principle, the relationship between the content of free water in aviation fuel and the color development of the chemical water meter can be established, thereby realizing intuitive detection of the content of free water in aviation fuels. The test paper for detecting free water in aviation fuels provided by the present invention can realize the color change process (when in contact with water) as yellow (0 ppm), few light yellowish-green spots (5 ppm), light yellowish-green spots (10 ppm), yellowish-green spots (20 ppm), green spots (30 ppm) and blue spots (40 ppm), is green when the content of free water is 30 ppm (critical value) and is blue when the content of free water is 40 ppm, has color development at low free water content (lower than 20 ppm, such as 10 ppm), and has obvious color change, fast and sensitive color development and easy judgment. At the critical value, the green is stable and is maintained for more than 24 hours under dry condition.

With respect to other ferric compounds such as ferric sulfate and other ferrous compounds such as ferrous chloride, the adoption of FeSO₄ by the present invention can be attributed to reasons as follows.
(1) FeSO₄, which also has relatively high stability at high temperature, is not susceptible to heat degeneration as the ferric compound such as ferric sulfate. Thus, it is possible to ensure that the test paper can be stored at high temperature, thereby reducing storage cost and improving its detection stability.
(2) FeSO₄ has high solubility in water, thus it possible to ensure that free water can fully participate in reaction during detection process, thereby improving detection accuracy and sensitivity.
(3) Unlike that the ferric compound such as ferric sulfate is hydrolyzed in contact with water to form ferric hydroxide precipitate coated on surface of the compound and retarding reaction, precipitate substance is not formed when FeSO₄ is dissolved in water, so that the detection sensitivity is ensured.

Although FeSO₄ has the advantages conducive to detection, it also has the problem that Fe²⁺ is easily oxidized into Fe³⁺ in air to interfere in the chemical reaction of equation (I). To solve the problem, a chelator is also added to FeSO₄ in the present invention, Fe³⁺ formed by oxidation of Fe²⁺ preferentially reacts with the chelator to form Fe³⁺ complex, thereby preventing Fe³⁺ from reacting with [Fe(CN)₆]³⁻, reducing influence of oxidation on the equation (I) and ensuring full reaction of Fe²⁺ and [Fe(CN)₆]³⁻.

The chelator includes one of ethylenediaminetetraacetic acid (EDTA), tartaric acid, tartrate salt, citric acid and citrate salt. Preferably, a weight ratio of FeSO₄ to the chelator is 1 : (0.2% - 1%) , more preferably 1 : (0.3 - 0.6 %) . Too low chelator content causes that oxidation-formed Fe³⁺ does not completely react with the chelator, thereby affecting reaction of Fe²⁺ and [Fe(CN)₆]³⁻ and resulting in a decrease in detection accuracy. While too high chelator content causes covering color development of the indigo product. Further, the chelator is citric acid or citrate salt. The combination of citric acid or citrate salt with FeSO₄ makes storage stability more excellent, thereby facilitating prolongation of storage time. More preferably, a weight ratio of FeSO₄ to the citric acid or citrate salt is 1 : 0.5% .

In accordance with the invention, a weight ratio of the K₃[Fe(CN)₆] to the powdery mixture of the FeSO₄ and the chelator is 1:(5 - 50). As known from the reaction equation (I), the amount of the FeSO₄ is amplified at the ratio to ensure that sufficient Fe²⁺ can participate in the reaction even if oxidation occurs. It reacts rapidly during the detection process. Meanwhile, excessive FeSO₄ improving the stability of Fe²⁺ so that the storage period is longer. More preferably, a weight ratio of the K₃[Fe(CN)₆] to the powdery mixture of the FeSO₄ and the chelator is 1:(11 - 22), most preferably, 1:15. Further and optimally, a weight ratio of the K₃[Fe(CN)₆] to the powdery mixture of the FeSO₄ and citric acid or potassium citrate is 1:15, and a weight ratio of the FeSO₄ to the citric acid or citrate salt is 1 : 0.5% .

Preferably, the loading amount of the powdery mixture of the FeSO₄ and the chelator on the filter paper is 0.5 - 5 mg/cm², more preferably 1.25 - 2.5 mg/cm². Too much loading of the powdery mixture of the FeSO₄ and the chelator can affect apparent color development of the indigo product, and too little loading thereof can result in too few amounts of reactant on per unit area of the test paper and inconspicuous color development due to too few amounts of indigo product. For loading manner, it is preferable to be coated by a brush, a cleansing cloth and an electrospray technique, and more preferably to be coat by spray-coating electrostatic powders. The ambient humidity during coating is controlled within 60%.

Preferably, the filter paper is one of an oil filter paper, a fast quantitative filter paper having pore size of 80 - 120 µm and a fast qualitative filter paper having pore size of 80 - 120 µm. The filter paper can improve detection efficiency and ensure detection sensitivity. Further, the FeSO₄ has a particle size of 20 - 50 µm. On one hand, the FeSO₄ with the particle size has good dispersibility on the filter paper and no agglomeration, thereby improving uniformity of its distribution on the surface of the filter paper; on the other hand, the efficiency of the reaction of FeSO₄ and K₃[Fe(CN)₆] is taken in consideration; and finally, the FeSO₄ with the particle size is most easily to realize electrostatic powder spray-coating, thereby facilitating industrial mass production.

More preferably, a fast quantitative filter paper having pore size of 100 µm or a 101 fast qualitative filter paper is adopted, the adoption of the test paper can realize fast detection within 2 minutes, and detection efficiency is improved. Cooperatively, the FeSO₄ has a particle size of 20 - 50 µm, more preferably 20 - 30 µm, and the powdery mixture of the FeSO₄ and the chelator is coated by electrostatic powder spray-coating.

In accordance with the invention, K₃[Fe(CN)₆] is loaded on the surface of the filter paper by soaking and drying, so that the distribution uniformity of K₃[Fe(CN)₆] on the surface of the filter paper can be improved, and light yellow test paper with uniform color distribution is formed, thereby improving detection accuracy and sensitivity.

Accordingly, a first aspect of the present invention provides a method for preparing a test paper to detect free water in aviation fuels, comprising the steps of: soaking a filter paper in an aqueous solution of K₃[Fe(CN)₆]; drying the filter paper to obtain an intermediate test paper; and coating a surface of the intermediate test paper with a powdery mixture of FeSO₄ and a chelator, wherein a weight ratio of the K3[Fe(CN)6] to the powdery mixture of the FeSO4 and the chelator is 1 : (5-50).

In the preparation process,
the step S1 is a step for loading K₃[Fe(CN)₆] on the filter paper. In the step, soaking-drying manner is adopted to improve distribution uniformity of K₃[Fe(CN)₆] on the surface of the filter paper and form light yellow test paper with uniform color distribution, thereby improving detection accuracy and sensitivity. In the step, a concentration of the aqueous solution of K3[Fe(CN)6] is preferably 3 - 10 g/L, and too low concentration of the aqueous solution of K₃[Fe(CN)₆] can cause too low content of the loaded K₃[Fe(CN)₆], and too few amounts of reaction product may cause inconspicuous color change, thus reducing detection sensitivity. Too high concentration of the aqueous solution of K₃[Fe(CN)₆] may cause the test paper to be too dark in color and may cause covering of the indigo reaction product in color, so that the reaction is unobvious and the detection sensitivity is affected. Further, when the test paper is soaked in the aqueous solution of K₃[Fe(CN)₆] with a concentration of 5 g/L and dried, the loading amount of K₃[Fe(CN)₆] on the test paper is optimal, that is, sufficient reactants are ensured without causing the test paper to be too dark in color. Still further, the filter paper is a fast quantitative filter paper having a pore size of 100 µm or a 101 fast qualitative filter paper.

The drying is used for removing water that adheres to the surface of the test paper in the soaking process. For selection of the drying temperature and time, the drying is performed at preferably 40 - 80°C for preferably 20 - 60 minutes. Too short drying time or too low temperature causes residual moisture in the test paper and further causes advanced color change after the powdery mixture of FeSO₄ and the chelator is coated, thereby reducing yield or accuracy of the detection result. Too high drying temperature or too long drying time causes excessive drying of the test paper, and the test paper becomes brittle or the color is too dark to affect color change. Most preferably, the drying is performed at 60°C for 30 minutes.

The step S2 is to coat the powdery mixture of the FeSO₄ and the chelator on the surface of the intermediate test paper. For coating manner, it is preferable to be coated by a brush, a cleansing cloth and an electrospray technique, and more preferably by electrostatic powder spray-coating. The ambient humidity during coating is controlled within 60%. Preferably, the powdery mixture of the FeSO₄ and the chelator used for coating is prepared by mixing the FeSO₄ and the chelator and milling the powdery mixture of the FeSO₄ and the chelator until the powdery mixture has a particle size of 20 - 50 µm. That the particle size of the FeSO₄ is controlled at the range has the advantages as follows: 1. FeSO₄ with the particle size has good dispersibility and excellent adhesion force on an oil filter paper, a fast quantitative filter paper having a pore size of 80 - 120 µm or a fast qualitative filter paper having a pore size of 80 - 120 µm and has no agglomeration, thereby improving uniformity of its distribution on the surface of the filter paper; 2. the efficiency of the reaction with of FeSO₄ and K₃[Fe(CN)₆] is taken in consideration; 3. the FeSO₄ with the particle size is most easily to realize electrostatic powder spray-coating, thereby facilitating industrial mass production. Most preferably, the step S2 includes mixing the FeSO₄ and the chelator and milling the powdery mixture of the FeSO₄ and the chelator until the powdery mixture has a particle size of 20 - 50 µm. The powdery mixture of the FeSO₄ and the chelator is coated on a surface of the intermediate test paper by electrostatic powder spray-coating. Milling of the FeSO₄ and the chelator together helps to improve mixing uniformity. Also, oxidation ratio of FeSO₄ is reduced during milling.

Preferably, the coating amount is 0.5 - 5 mg/cm² in the step. Too much coating of the powdery mixture of the FeSO₄ and the chelator may affect apparent color development of the indigo product, too little may result in too few amounts of reactant in per unit area of the test paper, thereby resulting in too little production of indigo product and unobvious color development. Further, in cooperation with electrostatic powder spray-coating, the coating amount of the powdery mixture of the FeSO₄ and the chelator is preferably 1.25 - 2.5 mg/cm², more preferably 1.65 mg/cm². The spray-coating amount is in favor of forming uniform and thickness-suitable coating.

The test paper for detecting free water in aviation fuels and preparation method thereof provided by the present invention has the following beneficial effects.
(1) The detection method is simple (only by suction-filtering to-be-detected aviation fuel, passing through the test paper and observing color change of the test paper), and has simple and easy operation, no need of specialization, no need of instrument, convenient use, naked eye intuitive and concise detection, fast detection and high efficiency.
(2) The test paper has long storage time and long effective service life (up to 15 months), is easy to be judged whether failure or not through intuitive color change. Detection reliability is improved.
(3) The test paper, which has light yellow base color, produces more obvious color change in contact with water for easy judgment.
(4) The test paper has high detection sensitivity, can realize the color change process (when in contact with water) as yellow (0 ppm), few light yellowish-green spots (5 ppm), light yellowish-green spots (10 ppm), yellowish-green spots (20 ppm), green spots (30 ppm) and blue spots (40 ppm), is green when the content of free water is 30 ppm (critical value) and is blue when the content of free water is 40 ppm, has color development at low free water content (lower than 20 ppm, such as 10 ppm), and has obvious color change, fast and sensitive color development and easy judgment. At the critical value, the green is stable and is maintained for more than 24 hours under dry condition.
(5) Raw materials are easily available, and the product has low cost.

Referring to Fig.1, another aspect of the present invention provides a device for detecting free water in aviation fuels, comprising: the test paper (30) prepared with the method as described above; a housing (10); a core sleeve (20) being inserted into the housing (10) to form a sealing connection; a cavity (12) formed between a front surface of the housing (10) and a front surface of the core sleeve (20); a suction hole (21) which passes through the core sleeve (20); and a fuel inlet (11) which passes through the front surface of the housing (10), wherein: the cavity (12) communicates with the suction hole (21) and with the fuel inlet (11); the test paper (30) is configured in the cavity (12); and the aviation fuel being tested passes by the test paper (30) along a fuel channel formed by the fuel inlet (11), the cavity (12) and the suction hole (21).

The detection of free water in aviation fuels with the device for detecting free water in aviation fuels provided in the embodiment comprises the operations as follows: installing a syringe or other sampling devices at end of the suction hole (21), fuel sample passing through the fuel hole (11), the test paper (30) and the suction hole (21) and entering the syringe in detection process, and observing color development of the test paper to determine whether the water content of the aviation fuel exceeds predetermined value.

Preferably, a diameter of the fuel hole (11) is 3 - 7 mm. Increase on the size of the fuel hole (11) is conducive to improving detection efficiency. Although increase on the size of the fuel hole (11) is conducive to improving detection speed, the contact area between the fuel being tested and the detection test paper is excessively large, resulting in inaccurate detection result. More preferably, a diameter of the fuel hole (11) is 4 - 5 mm.

Preferably, the housing (10) has transparent or translucent configuration to facilitate timely observation of color change of the test paper. Referring to FIG.1, a sealing connection structure with simple structure and convenient assembling is arranged between the housing (10) and the core sleeve (20), i.e., the sealing connection is formed by the inner wall of the housing (10) and the outer wall of the core sleeve (20) in tight engagement therewith. The inner wall of the housing (10) and the outer wall of the core sleeve (20) are matched annular conical surfaces. Further, in order to reduce assembling force and ensure sealing effect, the core sleeve (20) is provided with an annular inner groove (22) extending from the rear end thereof toward the front part, and the annular inner groove (22) is coaxially arranged with the suction hole (21).

In addition, in order to further prolong storage period, the device for detecting free water in aviation fuels is preferably provided with a sealing mechanism for sealing the fuel hole (11) and the suction hole (21). The sealing mechanism can be sealing plugs provided at the inlet of the fuel hole (11) and the outlet of the suction hole (21), or can be plastic sealing sleeves provided at the inlet of the fuel hole (11) and the outlet of the suction hole (21), or can be a plastic sealing sleeve provided on the whole outer surface of a water meter. The sealing mechanism can improve sealability of the cavity before application, so as to prevent oxidation of the FeSO₄ on the test paper therein. In application, the sealing mechanism is removed.

The device for detecting free water in aviation fuels has simple structure, simple and convenient assembling and simple and easy detection operation, is convenient for storage of the test paper as well, and is contributive to prolonging storage period.

The present invention is further specified by examples and comparative examples as follows.

### [Influence of Concentration of Aqueous Solution of K₃[Fe(CN)₆] on Test Paper]

### Examples 1-3 and comparative examples 1-2

The preparation process of the test paper comprises:
1. Adopting a fast quantitative filter paper having a pore size of 100 µm, and soaking in FeSO₄ solutions of different concentrations, wherein 200 pieces of the filter paper are soaked in the aqueous solution of K₃[Fe(CN)₆] 1 L, the area of each piece of the filter paper is 225 cm², and the concentrations of the aqueous solutions of K₃[Fe(CN)₆] used in the examples and the comparative examples are listed in Table 1;
2. Drying the filter paper at 60°C for 30 minutes to obtain an intermediate test paper;
3. Mixing FeSO₄ powder and citric acid at a weight ratio of 100:0.5, and milling the powdery mixture of the FeSO₄ and the chelator until the powdery mixture has a particle size of 20 µm; and
4. Coating the powdery mixture of the FeSO₄ and the chelator on a surface of the intermediate test paper by electrostatic powder spray-coating while controlling coating amount to be 1.65 mg/cm².

The aviation fuels with free water contents of 5 ppm, 10 ppm, 20 ppm, 30 ppm and 40 ppm are detected by the test paper, respectively. The detection is performed with the device shown in Fig.1. The diameter of the fuel hole is 5 mm, the volume of the fuel sample being tested is 5 mL, three repeated experiments are set, and the average value or the average value ± standard deviation of the three repeated experiments is applied as result. The detection results are listed in Table 1.

The contents in each cell of the left-most column in Tables 1-4 and Tables 6-9 respectively show example number, process parameter and test paper color.

**Table 1 Process Parameters and Detection Results in Examples 1-3 and Comparative Examples 1-2.**

| | **Detection result** | | **Detection time** |
|---|---|---|---|
| **Example 1** | 5 ppm | Few light yellowish-green spots, spot area/detection area≈5% | 65 - 70s |
| Concentration of aqueous solution of K₃[Fe(CN)₆]: 5 g/L | 10 ppm | Light yellowish-green spots, spot area/detection area≈40% | 65 - 70s |
| Light yellow test paper | 20 ppm | Yellowish-green spots, spot area/detection area≈60% | 65 - 70s |
| | 30 ppm | Green spots, spot area/detection area≈80% | 65 - 70s |
| | 40 ppm | Blue spots, spot area/detection area≈100% | 65 - 70s |
| **Example 2** | 5 ppm | Very few light yellowish-green spots, spot area/detection area≈1% | 100 - 105s |
| Concentration of aqueous solution of K₃[Fe(CN)₆]: 3 g/L | 10 ppm | Light yellowish-green spots, spot area/detection area≈20% | 100 - 105s |
| | 20 ppm | Yellowish-green spots, spot area/detection area≈40% | 100 - 105s |
| Light yellow test paper | 30 ppm | Green spots, spot area/detection area≈60% | 100 - 105s |
| | 40 ppm | Blue spots, spot area/detection area≈70% | 100 - 105s |
| **Example 3** | 5 ppm | Light yellowish-green spots, spot area/detection area≈10% | 60 - 65 s |
| Concentration of aqueous solution of K₃[Fe(CN)₆]: 10 g/L | 10 ppm | Light yellowish-green spots, spot area/detection area≈60% | 60 - 65 s |
| | 20 ppm | Yellowish-green spots, spot area/detection area≈80% | 60 - 65 s |
| Light yellow test paper | 30 ppm | Green spots, spot area/detection area≈90% | 60-65 s |
| | 40 ppm | Blue spots, spot area/detection area≈100% | 60 - 65 s |
| **Comparative Example 1** | 5 ppm | No visual change | - - |
| | 10 ppm | Very few light yellowish-green spots, spot area/detection area≈2% | 180 - 190 s |
| Concentration of aqueous solution of K₃[Fe(CN)₆]: 2 g/L | 20 ppm | Light yellowish-green spots, spot area/detection area≈8% | 180 - 190 s |
| | 30 ppm | Yellowish-green spots, spot area/detection area≈10% | 180 - 190 s |
| White yellowish test paper | 40 ppm | Green spots, spot area/detection area≈12% | 180 - 190 s |
| **Comparative Example 2** | 5 ppm | No visual change | - - |
| | 10 ppm | No visual change | - - |
| Concentration of aqueous solution of K₃[Fe(CN)₆]: 11 g/L | 20 ppm | Green spots, spot area/detection area≈50% | 60 - 65 s |
| | 30 ppm | Green spots, spot area/detection area≈90% | 60 - 65 s |
| Light orange test paper | 40 ppm | Blue spots, spot area/detection area≈100% | 60 - 65 s |

As shown in Table 1, the concentration of the aqueous solution of K₃[Fe(CN)₆] has influence on detection sensitivity of the test paper. If the concentration of the K₃[Fe(CN)₆] solution is too low, there is no detection effect in the case of extremely-low free water content (lower than 5 ppm, the same below), and the difference on the chromogenic reactions in the case of high free water content is unobvious and not easy to be visually distinguished. If the concentration of the aqueous solution of K₃[Fe(CN)₆] is too high, the color of the test paper is too dark, the color change cannot be visually observed in the case of extremely-low free water content, and it also has the problem that the difference on the chromogenic reactions in the case of high free water content is unobvious. The appropriate concentration of the aqueous solution of K₃[Fe(CN)₆] is 3 - 10 g/L. The test paper can realize the color change process (when in contact with water) as: yellow (0 ppm), few light yellowish-green spots (5 ppm), light yellowish-green spots (10 ppm), yellowish-green spots (20 ppm), green spots (30 ppm) and blue spots (40 ppm), is green when the content of free water is 30 ppm (critical value) and is blue when the content of free water is 40 ppm, has color development at low free water content (lower than 20 ppm, such as 10 ppm), has obvious color change, fast and sensitive color development and easy judgment, can realize detection within 2 minutes, and has high detection efficiency. Most preferably, the concentration is 5 g/L, where the difference on the chromogenic reactions of different free water contents is obvious and easy to be visually observed.

### [Influence of Filter Paper Selection on Test Paper]

### Examples 1, 4-5 and comparative example 3

The preparation process of the test paper comprises:
1. Adopting different filter papers, and soaking in aqueous solution of K₃[Fe(CN)₆] with a concentration of 5 g/L, wherein 200 pieces of the filter paper are soaked in the aqueous solution of K₃[Fe(CN)₆] 1 L, the area of each piece of the filter paper is 225 cm², and the types of the filter paper used in the examples and the comparative example are listed in Table 2;
2. Drying the filter paper at 60°C for 30 minutes to obtain intermediate test paper;
3. Mixing FeSO₄ powder and citric acid at a weight ratio of 100:0.5, and milling the powdery mixture of the FeSO₄ and the chelator until the powdery mixture has a particle size of 20 µm; and
4. Coating a surface of the intermediate test paper with the powdery mixture of the FeSO₄ and the chelator by electrostatic powder spray-coating while controlling coating amount to be 1.65 mg/cm².

The aviation fuels with free water contents of 5 ppm, 10 ppm, 20 ppm, 30 ppm and 40 ppm are detected by the test paper, respectively. The detection is performed with the device shown in Fig.1. The diameter of the fuel hole is 5 mm, the volume of the fuel sample being tested is 5 mL, three repeated experiments are set, and the average value or the average value ± standard deviation of the three repeated experiments is applied as result. The detection results are listed in Table 2.

**Table 2 Process Parameters and Detection Results in Examples 1, 4-5 and Comparative Example 3**

| | **Detection result** | | **Detection time** |
|---|---|---|---|
| **Example 1** | 5 ppm | Few light yellowish-green spots, spot area/detection area≈5% | 65 - 70s |
| Fast quantitative filter paper having a pore size of 100 µm | 10 ppm | Light yellowish-green spots, spot area/detection area≈40% | 65 - 70s |
| Light yellow test paper | 20 ppm | Yellowish-green spots, spot area/detection area≈60% | 65 - 70 s |
| | 30 ppm | Green spots, spot area/detection area≈80% | 65 - 70s |
| | 40 ppm | Blue spots, spot area/detection area≈100% | 65 - 70s |
| **Example 4** | 5 ppm | Few light yellowish-green spots, spot area/detection area≈5% | 65 - 70s |
| 101 Fast qualitative filter paper | 10 ppm | Light yellowish-green spots, spot area/detection area≈40% | 65 - 70s |
| Light yellow test paper | 20 ppm | Yellowish-green spots, spot area/detection area≈60% | 65 - 70s |
| | 30 ppm | Green spots, spot area/detection area≈80% | 65 - 70s |
| | 40 ppm | Blue spots, spot area/detection area≈100% | 65 - 70s |
| **Example 5** | 5 ppm | Few light yellowish-green spots, spot area/detection area≈5% | 65 - 70s |
| Fast quantitative filter paper having a pore size of 80 µm | 10 ppm | Light yellowish-green spots, spot area/detection area≈40% | 65 - 70s |
| | 20 ppm | Yellowish-green spots, spot area/detection area≈50% | 65 - 70s |
| Light yellow test paper | 30 ppm | Green spots, spot area/detection area≈80% | 65 - 70s |
| | 40 ppm | Blue spots, spot area/detection area≈100% | 65 - 70 s |
| **Comparative Example 6** | 5 ppm | No visual change | - - |
| | 10 ppm | Light yellowish-green spots, spot area/detection area≈20% | 85 - 90s |
| Fast qualitative filter paper having pore size of 120 µm | 20 ppm | Yellowish-green spots, spot area/detection area≈50% | 85 - 90s |
| | 30 ppm | Green spots, spot area/detection area≈70% | 85 - 90s |
| White yellowish test paper | 40 ppm | Blue spots, spot area/detection area≈80% | 85 - 90s |
| **Comparative Example 3** | 5 ppm | No visual change | - - |
| | 10 ppm | Light yellowish-green spots, spot area/detection area≈20% | 290 - 300 s |
| Medium-speed quantitative filter paper having a pore size of 50 µm | 20 ppm | Yellowish-green spots, spot area/detection area≈40% | 290 - 300 s |
| | 30 ppm | Green spots, spot area/detection area≈60% | 290 - 300 s |
| Light yellow test paper | 40 ppm | Blue spots, spot area/detection area≈70% | 290 - 300 s |

As shown in Table 2, the selection of the filter paper has influence on detection sensitivity. If the pore size of the filter paper is too small, the detection time is too long, there is no detection effect in the case of extremely low free-water content, and the difference on the chromogenic reactions in the case of high free water content is unobvious and not easy to be visually distinguished. Preferably, the filter paper employs one of a fast quantitative filter paper having a pore size of 80 - 120 µm, a fast qualitative filter paper having a pore size of 80 - 120 µm and an oil filter paper having equivalent properties. The test paper can realize the color change process (when in contact with water) as: yellow (0 ppm), few light yellowish-green spots (5 ppm), light yellowish-green spots (10 ppm), yellowish-green spots (20 ppm), green spots (30 ppm) and blue spots (40 ppm), is green when the content of free water is 30 ppm (critical value) and is blue when the content of free water is 40 ppm, has color development at low free water content (lower than 20 ppm, such as 10 ppm), has obvious color change, fast and sensitive color development and easy judgment, can realize detection within 2 minutes, and has high detection efficiency. Most preferably, fast quantitative filter paper with pore size of 100 µm or 101 fast qualitative filter paper is employed, where the difference on the chromogenic reactions of different free water contents is obvious and easy to be visually observed.

### [Influence of Drying on Test Paper]

### Examples 1, 7-8 and comparative examples 4-5

1. A fast quantitative filter paper having a pore size of 100 µm is adopted and soaked in aqueous solution of K₃[Fe(CN)₆] with a concentration of 5 g/L, wherein 200 pieces of the filter paper are soaked in the aqueous solution of K₃[Fe(CN)₆] 1 L, and the area of each piece of the filter paper is 225 cm².
2. Drying is performed to the filter paper to obtain intermediate test paper, wherein different drying temperatures and times are set, and the drying temperatures and times in the examples and comparative examples are listed in Table 3.
3. FeSO₄ powder and citric acid are mixed at weight ratio of 100:0.5, and milling is performed to the powdery mixture of the FeSO₄ and the chelator until the powdery mixture has a particle size of 20 µm.
4. The powdery mixture of the FeSO₄ and the chelator is coated on a surface of the intermediate test paper by electrostatic powder spray-coating while controlling coating amount to be 1.65 mg/cm².

The aviation fuels with free water contents of 5 ppm, 10 ppm, 20 ppm, 30 ppm and 40 ppm are detected by the test paper, respectively. The detection is performed with the device shown in Fig.1. The diameter of the fuel hole is 5 mm, the volume of the fuel sample being tested is 5 mL, three repeated experiments are set, and the average value or the average value±standard deviation of the three repeated experiments is applied as result. The detection results are listed in Table 3.

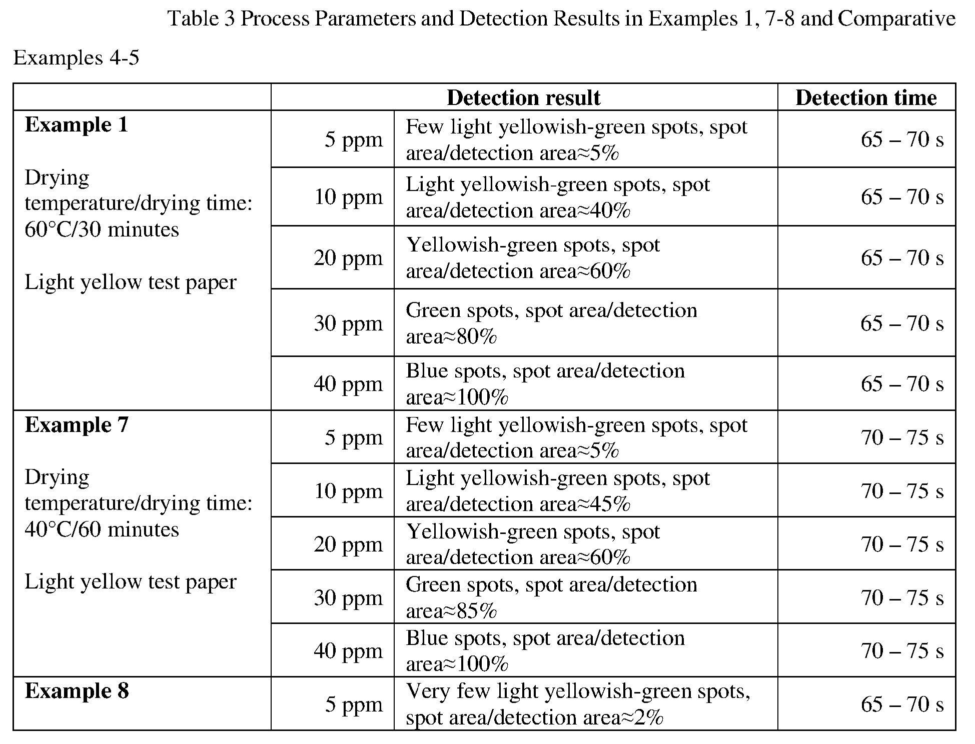

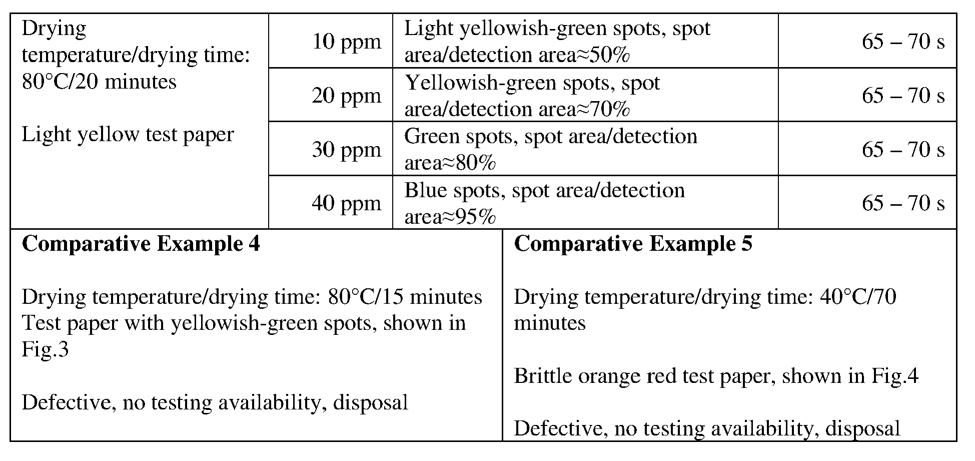

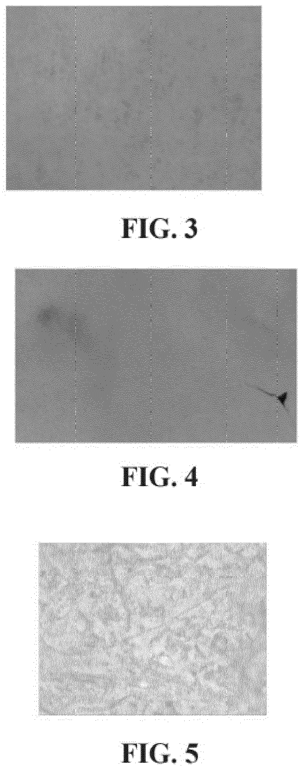

As shown in Table 3 and Figs. 3-4, drying temperature and time have influence on yield. If the drying temperature is too low or the drying time is too short, the test paper may change color in advanced. If the drying temperature is too high or the drying time is too long, the test paper becomes too dark in color and becomes brittle. The test paper in both cases is defective and is ineffective for detection operation. Preferably, drying is performed at 40 - 80°C for 20 - 60 minutes, and the prepared test paper is light yellow. The test paper can realize the color change process (when in contact with water) as: yellow (0 ppm), few light yellowish-green spots (5 ppm), light yellowish-green spots (10 ppm), yellowish-green spots (20 ppm), green spots (30 ppm) and blue spots (40 ppm), is green when the content of free water is 30 ppm (critical value) and is blue when the content of free water is 40 ppm, has color development at low free water content (lower than 20 ppm, such as 10 ppm), has obvious color change, fast and sensitive color development and easy judgment, can realize detection within 2 minutes, and has high detection efficiency. Most preferably, the drying temperature is 60°C, and the drying time is 30 minutes, where the test paper has optimal color after drying, and the difference on the chromogenic reactions of different free water contents is obvious and easy to be visually observed.

### [Influence of Chelator on Test Paper]

### Examples 1, 10-11 and comparative example 6

1. A fast quantitative filter paper having a pore size of 100 µm is adopted and soaked in aqueous solution of K₃[Fe(CN)₆] with a concentration of 5 g/L, wherein 200 pieces of the filter paper are soaked in the aqueous solution of K₃[Fe(CN)₆] 1 L, and the area of each piece of the filter paper is 225 cm².
2. Drying is performed to the filter paper at 60°C for 30 minutes to obtain intermediate test paper.
3. FeSO₄ powder and a chelator are mixed at a weight ratio of 100:0.5, and milling is performed to the powdery mixture of the FeSO₄ and the chelator until the powdery mixture has a particle size of 20 µm, wherein the chelators used in the examples and the comparative example are listed in Table 4 (no chelator in the comparative example).
4. The powdery mixture of the FeSO₄ and the chelator is coated on a surface of the intermediate test paper by electrostatic powder spray-coating while controlling coating amount to be 1.65 mg/cm².

The aviation fuels with free water contents of 5 ppm, 10 ppm, 20 ppm, 30 ppm and 40 ppm are detected by the test paper, respectively. The detection is performed with the device shown in Fig.1. The diameter of the fuel hole is 5 mm, the volume of the fuel sample being tested is 5 mL, three repeated experiments are set, and the average value or the average value±standard deviation of the three repeated experiments is applied as result. The detection results are listed in Table 4.

**Table 4 Process Parameters and Detection Results in Examples 1, 10-11 and Comparative Example 6**

| | **Detection result** | | **Detection time** |
|---|---|---|---|
| **Example 1** | 5 ppm | Few light yellowish-green spots, spot area/detection area≈5% | 65 - 70s |
| Chelator: citric acid | 10 ppm | Light yellowish-green spots, spot area/detection area≈40% | 65 - 70s |
| Light yellow test paper | 20 ppm | Yellowish-green spots, spot area/detection area≈60% | 65 - 70s |
| | 30 ppm | Green spots, spot area/detection area≈80% | 65 - 70s |
| | 40 ppm | Blue spots, spot area/detection area≈100% | 65 - 70 s |
| **Example 10** | 5 ppm | Few light yellowish-green spots, spot area/detection area≈5% | 65 - 70s |
| Chelator: sodium citrate | 10 ppm | Light yellowish-green spots, spot area/detection area≈40% | 65 - 70s |
| Light yellow test paper | 20 ppm | Yellowish-green spots, spot area/detection area≈60% | 65 - 70s |
| | 30 ppm | Green spots, spot area/detection area≈80% | 65 - 70s |
| | 40 ppm | Blue spots, spot area/detection area≈100% | 65 - 70 s |
| **Example 11** | 5 ppm | Few light yellowish-green spots, spot area/detection area≈5% | 90 - 95 s |
| Chelator: EDTA | 10 ppm | Light yellowish-green spots, spot area/detection area≈50% | 90 - 95 s |
| Light yellow test paper | 20 ppm | Yellowish-green spots, spot area/detection area≈60% | 90 - 95 s |
| | 30 ppm | Green spots, spot area/detection area≈85% | 90 - 95 s |
| | 40 ppm | Blue spots, spot area/detection area≈100% | 90 - 95 s |
| **Comparative Example 6** | 5 ppm | Few light yellowish-green spots, spot area/detection area≈2% | 150 - 160 s |
| No chelator | 10 ppm | Light yellowish-green spots, spot area/detection area≈25% | 150 - 160 s |
| Light yellow test paper | 20 ppm | Yellowish-green spots, spot area/detection area≈40% | 150 - 160 s |
| | 30 ppm | Green spots, spot area/detection area≈55% | 150 - 160 s |
| | 40 ppm | Blue spots, spot area/detection area≈60% | 150 - 160 s |

100 pieces of the test paper prepared in each of the examples 1 and 10 and the comparative example 6 are placed in the device as shown in Fig.1, and then stored at room temperature to perform storage period test. The failure standard for the test paper is that 2 pieces of the test paper from 100 samples being tested have yellow-green spots. The detection results are listed in Table 5.

**Table 5 Detection Results of Storage Period**

| | **Example 1** | **Example 10** | **Example 11** | **Comparative Example 6** |
|---|---|---|---|---|
| Storage period | 15 months | 15 months | 12 months | 8 months |

As shown in Table 4 and Table 5, the addition of the chelator helps to improve detection sensitivity and storage period of the test paper, and the type of the chelator also has influence on storage period of the test paper. The use of citric acid or citrate salt helps to improve performance stability of the test paper and prolong storage period up to 15 months.

### [Influence of Particle Size of FeSO₄ on Test Paper]

### Examples 1, 12-13 and comparative examples 7-8

1. A fast quantitative filter paper having a pore size of 100 µm is adopted and soaked in aqueous solution of K₃[Fe(CN)₆] with a concentration of 5 g/L, wherein 200 pieces of the filter paper are soaked in the aqueous solution of K₃[Fe(CN)₆] 1 L, and the area of each piece of the filter paper is 225 cm².
2. Drying is performed to the filter paper at 60°C for 30 minutes to obtain intermediate test paper.
3. FeSO₄ powder and citric acid are mixed at a weight ratio of 100:0.5, and milling is performed to the powdery mixture of the FeSO₄ and the chelator until the powdery mixture has a particle size as being listed in Table 6.
4. The powdery mixture of the FeSO₄ and the chelator is coated on a surface of the intermediate test paper by electrostatic powder spray-coating while controlling coating amount to be 1.65 mg/cm².

The aviation fuels with free water contents of 5 ppm, 10 ppm, 20 ppm, 30 ppm and 40 ppm are detected by the test paper, respectively. The detection is performed with the device shown in Fig.1. The diameter of the fuel hole is 5 mm, the volume of the fuel sample being tested is 5 mL, three repeated experiments are set, and the average value or the average value ± standard deviation of the three repeated experiments is applied as result. The detection results are listed in Table 6. The detection results of the test paper prepared in example 1 are listed in Fig.2.

**Table 6 Process Parameters and Detection Results in Examples 1,12-13 and Comparative Examples 7-8**

| | **Detection result** | | **Detection time** |
|---|---|---|---|
| **Example 1** | 5 ppm | Few light yellowish-green spots, spot area/detection area≈5% | 65 - 70s |
| Particle size of FeSO₄: 20 µm | 10 ppm | Light yellowish-green spots, spot area/detection area≈40% | 65 - 70s |
| Light yellow test paper | 20 ppm | Yellowish-green spots, spot area/detection area≈60% | 65 - 70s |
| | 30 ppm | Green spots, spot area/detection area≈80% | 65 - 70s |
| | 40 ppm | Blue spots, spot area/detection area≈100% | 65 - 70 s |
| **Example 12** | 5 ppm | Light yellowish-green spots, spot area/detection area≈10% | 95 - 100 s |
| Particle size of FeSO₄: 50 µm | 10 ppm | Light yellowish-green spots, spot area/detection area≈60% | 95 - 100 s |
| Light yellow test paper | 20 ppm | Yellowish-green spots, spot area/detection area≈70% | 95 - 100 s |
| | 30 ppm | Green spots, spot area/detection area≈90% | 95 - 100 s |
| | 40 ppm | Blue spots, spot area/detection area≈100% | 95 - 100 s |
| **Example 13** | 5 ppm | Few light yellowish-green spots, spot area/detection area≈5% | 65 - 70s |
| Particle size of FeSO₄: 30 µm | 10 ppm | Light yellowish-green spots, spot area/detection area≈50% | 65 - 70s |
| Light yellow test paper | 20 ppm | Yellowish-green spots, spot area/detection area≈70% | 65 - 70s |
| | 30 ppm | Green spots, spot area/detection area≈85% | 65 - 70s |
| | 40 ppm | Blue spots, spot area/detection area≈100% | 65 - 70s |
| **Comparative Example 7** | 5 ppm | Very few light yellowish-green spots, spot area/detection area≈1% | 60 - 65 s |
| Particle size of FeSO₄: 10 µm | 10 ppm | Light yellowish-green spots, spot area/detection area≈30% | 60 - 65 s |
| | | Yellowish-green spots, spot area/detection area≈10% | |
| White yellowish test paper | 20 ppm | Yellowish-green spots, spot area/detection area≈50% | 60 - 65 s |
| | | Green spots, spot area/detection area≈20% | |
| | 30 ppm | Green spots, spot area/detection area≈70% | 60 - 65 s |
| | | Blue spots, spot area/detection area≈15% | |
| | 40 ppm | Blue spots, spot area/detection area≈90% | 60 - 65 s |
| | | Green spots, spot area/detection area≈10% | |
| **Comparative Example 8** | 5 ppm | No visual change | - - |
| Particle size of FeSO₄: 60 µm | 10 ppm | Light yellowish-green spots, spot area/detection area≈30% | 180 - 190 s |
| | | | |
| | 20 ppm | Yellowish-green spots, spot area/detection area≈45% | 180 - 190 s |
| Light orange test paper | 30 ppm | Green spots, spot area/detection area≈60% | 180 - 190 s |
| | 40 ppm | Blue spots, spot area/detection area≈70% | 180 - 190 s |

As shown in Table 6, the particle size of the FeSO₄ has influence on uniformity of its distribution on the test paper, thus influencing detection sensitivity. If the particle size is too small, it is easy to agglomerate, causing uneven distribution on the test paper and poor adhesion, further resulting in the occurrence of various color change areas during detection. If the particle size is too large, the detection sensitivity is reduced, and the difference on the chromogenic reactions of different free water contents is not easy to be visually observed. Preferably, the FeSO₄ has a particle size of 20 - 50 µm, and more preferably, 20 - 30 µm. The test paper can realize the color change process (when in contact with water) as: yellow (0 ppm), few light yellowish-green spots (5 ppm), light yellowish-green spots (10 ppm), yellowish-green spots (20 ppm), green spots (30 ppm) and blue spots (40 ppm), is green when the content of free water is 30 ppm (critical value) and is blue when the content of free water is 40 ppm, has color development at low free water content (lower than 20 ppm, such as 10 ppm), has obvious color change, fast and sensitive color development and easy judgment, can realize detection within 2 minutes, and has high detection efficiency. Most preferably, the particle size of the FeSO₄ is 20 µm, where the uniformity of its distribution on the surface of the test paper are highest, and the difference on the chromogenic reactions of different free water contents is obvious and easy to be visually observed.

### [Influence of Weight Ratio of Chelator to FeSO₄ to on Test Paper]

### Examples 1, 12-13 and comparative examples 7-8

1. A fast quantitative filter paper having a pore size of 100 µm is adopted and soaked in aqueous solution of K₃[Fe(CN)₆] with a concentration of 5 g/L, wherein 200 pieces of the filter paper are soaked in the aqueous solution of K₃[Fe(CN)₆] 1 L, and the area of each piece of the filter paper is 225 cm².
2. Drying is performed to the filter paper at 60°C for 30 minutes to obtain intermediate test paper.
3. FeSO₄ powder and citric acid are mixed at different weight ratios, and milling is performed to obtain powdery mixture of the FeSO₄ and the chelator, wherein the weight ratio of the FeSO₄ to citric acid in the examples and comparative examples are listed in Table 7.
4. The powdery mixture of the FeSO₄ and the chelator is coated on a surface of the intermediate test paper by electrostatic powder spray-coating while controlling coating amount to be 1.65 mg/cm².

The aviation fuels with free water contents of 5 ppm, 10 ppm, 20 ppm, 30 ppm and 40 ppm are detected by the test paper, respectively. The detection is performed with the device shown in Fig.1. The diameter of the fuel hole is 5 mm, the volume of the fuel sample being tested is 5 mL, three repeated experiments are set, and the average value or the average value±standard deviation of the three repeated experiments is applied as result. The detection results are listed in Table 7.

**Table 7 Process Parameters and Detection Results in Examples 1, 11-15 and Comparative Examples 9-10**

| | **Detection result** | | **Detection time** |
|---|---|---|---|
| **Example 1** | 5 ppm | Few light yellowish-green spots, spot area/detection area≈5% | 65 - 70s |
| M_{citric acid}:M _{FeSO4}=100:0.5 | 10 ppm | Light yellowish-green spots, spot area/detection area≈40% | 65 - 70s |
| Light yellow test paper | 20 ppm | Yellowish-green spots, spot area/detection area≈60% | 65 - 70s |
| | 30 ppm | Green spots, spot area/detection area≈80% | 65 - 70s |
| | 40 ppm | Blue spots, spot area/detection area≈100% | 65 - 70 s |
| **Example 14** | 5 ppm | Few light yellowish-green spots, spot area/detection area≈5% | 95 - 100 s |
| M_{citric acid}:M _{FeSO4}=100:0.3 | 10 ppm | Light yellowish-green spots, spot area/detection area≈20% | 95 - 100 s |
| Light yellow test paper | 20 ppm | Yellowish-green spots, spot area/detection area≈40% | 95 - 100 s |
| | 30 ppm | Green spots, spot area/detection area≈55% | 95 - 100 s |
| | 40 ppm | Blue spots, spot area/detection area≈95% | 95 - 100 s |
| **Example 15** | 5 ppm | Few light yellowish-green spots, spot area/detection area≈5% | 85 - 90s |
| M_{citric acid}:M _{FeSO4}=100:0.6 | 10 ppm | Light yellowish-green spots, spot area/detection area≈60% | 85 - 90s |
| Light yellow test paper | 20 ppm | Yellowish-green spots, spot area/detection area≈70% | 85 - 90s |
| | 30 ppm | Green spots, spot area/detection area≈85% | 85 - 90s |
| | 40 ppm | Blue spots, spot area/detection area≈100% | 85 - 90s |
| **Comparative Example 9** | 5 ppm | Very few light yellowish-green spots, spot area/detection area≈2% | 180 - 190 s |
| M_{citric acid}:M _{FeSO4}=100:0.1 | 10 ppm | Light yellowish-green spots, spot area/detection area≈30% | 180 - 190 s |
| Light yellow test paper | 20 ppm | Yellowish-green spots, spot area/detection area≈45% | 180 - 190 s |
| | 30 ppm | Green spots, spot area/detection area≈60% | 180 - 190 s |
| | 40 ppm | Blue spots, spot area/detection area≈65% | 180 - 190 s |
| **Comparative Example 10** | 5 ppm | No visual change | - - |
| | 10 ppm | Light yellowish-green spots, spot area/detection area≈30% | 90 - 100 s |
| M_{citric acid}:M _{FeSO4}=100:1.2 | 20 ppm | Green spots, spot area/detection area≈55% | 90 - 100 s |
| White yellowish test paper | 30 ppm | Green spots, spot area/detection area≈90% | 90 - 100 s |
| | 40 ppm | Blue spots, spot area/detection area≈100% | 90 - 100 s |

As shown in Table 7, the weight ratio of the chelator to the FeSO₄ has influence on detection sensitivity of the test paper. If the ratio of the chelator is too low, the detection sensitivity is low, and the difference on the chromogenic reactions of different free water contents is not easy to be visually observed. If the ratio is too high, the detection of low water content is ineffective, and the detection sensitivity is low. Preferably, a weight ratio of the FeSO₄ to the chelator is 1 : (0.3% - 0.6%) . The test paper can realize the color change process (when in contact with water) as: yellow (0 ppm), few light yellowish-green spots (5 ppm), light yellowish-green spots (10 ppm), yellowish-green spots (20 ppm), green spots (30 ppm) and blue spots (40 ppm), is green when the content of free water is 30 ppm (critical value) and is blue when the content of free water is 40 ppm, has color development at low free water content (lower than 20 ppm, such as 10 ppm), has obvious color change, fast and sensitive color development and easy judgment, can realize detection within 2 minutes, and has high detection efficiency. Most preferably, the chelator accounts for 0.5 wt% of the FeSO₄, where the difference on the chromogenic reactions of different free water contents is obvious and is easy to be visually observed.

### [Influence of Coating Amount on Test Paper]

### Examples 1, 16-17 and comparative examples 11-12

The preparation process of the test paper comprises:
1. Adopting a fast quantitative filter paper having a pore size of 100 µm, and soaking in aqueous solution of K₃[Fe(CN)₆] with a concentration of 5 g/L, wherein 200 pieces of the filter paper are soaked in the aqueous solution of K₃[Fe(CN)₆] 1 L, and the area of each piece of the filter paper is 225 cm²;
2. Drying the filter paper at 60°C for 30 minutes to obtain intermediate test paper;
3. Mixing FeSO₄ powder and citric acid at a weight ratio of 100:0.5, and milling the powdery mixture of the FeSO₄ and the chelator until the powdery mixture has a particle size of 20 µm; and
4. Coating a surface of the intermediate test paper with the powdery mixture of the FeSO₄ and the chelator by electrostatic powder spray-coating, wherein the coating amount in the examples and the comparative examples are listed in Table 2.

The aviation fuels with free water contents of 5 ppm, 10 ppm, 20 ppm, 30 ppm and 40 ppm are detected by the test paper, respectively. The detection is performed with the device shown in Fig.1. The diameter of the fuel hole is 5 mm, the volume of the fuel sample being tested is 5 mL, three repeated experiments are set, and the average value or the average value ± standard deviation of the three repeated experiments is applied as result. The detection results are listed in Table 8.

**Table 8 Process Parameters and Detection Results in Examples 1, 11-15 and Comparative Examples 9-10**

| | **Detection result** | | **Detection time** |
|---|---|---|---|
| **Example 1** | 5 ppm | Few light yellowish-green spots, spot area/detection area≈5% | 65 - 70s |
| Coating amount: 1.65 mg/cm² | 10 ppm | Light yellowish-green spots, spot area/detection area≈40% | 65 - 70s |
| Light yellow test paper, SEM photograph shown in Fig.5 | 20 ppm | Yellowish-green spots, spot area/detection area≈60% | 65 - 70s |
| | 30 ppm | Green spots, spot area/detection area≈80% | 65 - 70s |
| | 40 ppm | Blue spots, spot area/detection area≈100% | 65 - 70 s |
| **Example 16** | 5 ppm | Very few light yellowish-green spots, spot area/detection area≈1% | 65 - 70s |
| Coating amount: 1.25 mg/cm² | 10 ppm | Light yellowish-green spots, spot area/detection area≈30% | 65 - 70s |
| Light yellow test paper | 20 ppm | Yellowish-green spots, spot area/detection area≈50% | 65 - 70s |
| | 30 ppm | Green spots, spot area/detection area≈80% | 65 - 70s |
| | 40 ppm | Blue spots, spot area/detection area≈100% | 65 - 70 s |
| **Example 17** | 5 ppm | Light yellowish-green spots, spot area/detection area≈10% | 110 - 120 s |
| Coating amount: 2.5 mg/cm² | 10 ppm | Light yellowish-green spots, spot area/detection area≈50% | 110 - 120 s |
| Light yellow test paper | 20 ppm | Yellowish-green spots, spot area/detection area≈70% | 110 - 120 s |
| | 30 ppm | Green spots, spot area/detection area≈85% | 110 - 120 s |
| | 40 ppm | Blue spots, spot area/detection area≈100% | 110 - 120 s |
| **Comparative Example 11** | 5 ppm | No visual change | - - |
| | 10 ppm | Few light yellowish-green spots, spot area/detection area≈5% | 60 - 65 s |
| Coating amount: 1 mg/cm² | 20 ppm | Light yellowish-green spots, spot area/detection area≈20% | 60 - 65 s |
| Light yellow test paper, SEM photograph shown in Fig.6 | 30 ppm | Yellowish-green spots, spot area/detection area≈35% | 60 - 65 s |
| | 40 ppm | Green spots, spot area/detection area≈50% | 60 - 65 s |
| **Comparative Example 12** | 5 ppm | Light yellowish-green spots, spot area/detection area≈10% | 180 - 190 s |
| Coating amount: 2.7 mg/cm² | 10 ppm | Light yellowish-green spots, spot area/detection area≈30% | 180 - 190 s |
| | | Yellowish-green spots, spot area/detection area≈5% | |
| Light yellow test paper, SEM photograph shown in Fig.7 | 20 ppm | Yellowish-green spots, spot area/detection area≈50% | 180 - 190 s |
| | | Green spots, spot area/detection area≈15% | |
| | 30 ppm | Green spots, spot area/detection area≈70% | 180 - 190 s |
| | | Blue spots, spot area/detection area≈10% | |
| | 40 ppm | Blue spots, spot area/detection area≈80% | 180 - 190 s |
| | | Green spots, spot area/detection area≈20% | |

As shown in Table 8 and Figs.5-7, the coating amount has influence on detection sensitivity of the test paper. If the coating amount is too small, there is no detection effect in the case of extremely low free-water content, the difference on the chromogenic reactions in the case of high free water content is unobvious and not easy to be visually distinguished, and the detection sensitivity is low. If the coating amount is too high, uneven distribution on the test paper may be caused, resulting in occurrence of various color change areas in the detection process and further affecting accuracy of the detection results. Preferably, the coating amount is 1.25-2.5 mg/cm². The test paper can realize the color change process (when in contact with water) as: yellow (0 ppm), few light yellowish-green spots (5 ppm), light yellowish-green spots (10 ppm), yellowish-green spots (20 ppm), green spots (30 ppm) and blue spots (40 ppm), is green when the content of free water is 30 ppm (critical value) and is blue when the content of free water is 40 ppm, has color development at low free water content (lower than 20 ppm, such as 10 ppm), has obvious color change, fast and sensitive color development and easy judgment, can realize detection within 2 minutes, and has high detection efficiency. Most preferably, the coating amount is 1.65 mg/cm², where the difference on the chromogenic reactions of different free water contents is obvious and easy to be visually observed.

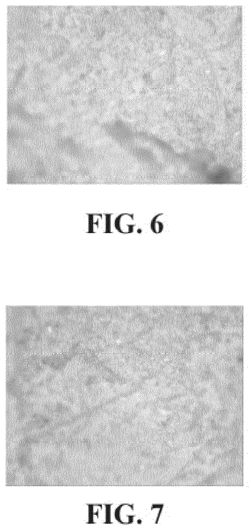

As observed in Figs.5-7, compared with Fig.6 and Fig.7, the stripe distribution in Fig.5 is uniform, the coating uniformity is good, the stripe distributions in Fig.6 and Fig.7 are non-uniform, and the stripe local distribution is over-crowded. The coating uniformity and the coating amount have influence on product detection sensitivity. Under a certain amount, the more uniform the color development effect is, the higher the sensitivity is. The free water in the to-be-detected object is often unevenly distributed, and in actual detection, if the coating amount is too small or the coating is non-uniform and cannot be directly contacted with the free water, the color development of the product is affected, so that the detection accuracy is low. If the coating amount is excessively large and the coating is stacked on the test paper, the product and free water do not completely react, resulting in influence on color development effect or color uniformity and also resulting in direct influence on detection time.

### [Effect of Weight Ratio of K₃[Fe(CN)₆] to Powdery Mixture of FeSO₄ and Chelator on Test Paper]

### Examples 1, 18-19 and comparative examples 13-14

The preparation process of the test paper comprises:
1. Adopting a fast quantitative filter paper having a pore size of 100 µm, and soaking in aqueous solution of K₃[Fe(CN)₆] with a concentration of 5 g/L, wherein different pieces of the filter paper are soaked in the aqueous solution of K₃[Fe(CN)₆] 1 L to control distribution amount of the K₃[Fe(CN)₆] on single piece of the filter paper, the area of each piece of the filter paper is 225 cm², and the distribution amounts of the K₃[Fe(CN)₆] on single piece of the filter paper in the examples and the comparative examples are listed in Table 9;
2. Drying the filter paper at 60°C for 30 minutes to obtain intermediate test paper;
3. Mixing FeSO₄ powder and citric acid at a weight ratio of 100:0.5, and milling the powdery mixture of the FeSO₄ and the chelator until the powdery mixture has a particle size of 20 µm; and
4. Coating a surface of the intermediate test paper with the powdery mixture of the FeSO₄ and citric acid by electrostatic powder spray-coating while controlling coating amount to be 1.65 mg/cm².

The aviation fuels with free water contents of 5 ppm, 10 ppm, 20 ppm, 30 ppm and 40 ppm are detected by the test paper, respectively. The detection is performed with the device shown in Fig.1. The diameter of the fuel hole is 5 mm, the volume of the fuel sample being tested is 5 mL, three repeated experiments are set, and the average value or the average value ± standard deviation of the three repeated experiments is applied as result. The detection results are listed in Table 9.

**Table 9 Process Parameters and Detection Results in Examples 1, 18-19 and Comparative Examples 13-14**

| | **Detection result** | | **Detection time** |
|---|---|---|---|
| **Example** 1 | 5 ppm | Few light yellowish-green spots, spot area/detection area≈5% | 65 - 70s |
| Distribution amount of K₃[Fe(CN)₆ on test paper: 0.11 mg/cm² | 10 ppm | Light yellowish-green spots, spot area/detection area≈40% | 65 - 70s |
| M_{K3[Fe(CN)6]}:M_{(FeSO4+citric acid)}=1:15 | 20 ppm | Yellowish-green spots, spot area/detection area≈60% | 65 - 70s |
| Light yellow test paper | 30 ppm | Green spots, spot area/detection area≈80% | 65 - 70s |
| | 40 ppm | Blue spots, spot area/detection area≈100% | 65 - 70s |
| **Example 8** | 5 ppm | Few light yellowish-green spots, spot area/detection area≈5% | 65 - 70s |
| Distribution amount of K₃[Fe(CN)₆ on test paper: 0.15 mg/cm² | 10 ppm | Light yellowish-green spots, spot area/detection area≈40% | 65 - 70s |
| M_{K3[Fe(CN)6]}:M_{(FeSO4+citricacid)}=1:11 | 20 ppm | Yellowish-green spots, spot area/detection area≈60% | 65 - 70s |
| Light yellow test paper | 30 ppm | Green spots, spot area/detection area≈80% | 65 - 70s |
| | 40 ppm | Blue spots, spot area/detection area≈100% | 65 - 70 s |
| **Example 19** | 5 ppm | Light yellowish-green spots, spot area/detection area≈10% | 95 - 100 s |
| Distribution amount of K₃[Fe(CN)₆ on test paper: 0.075 mg/cm² | 10 ppm | Light yellowish-green spots, spot area/detection area≈45% | 95 - 100 s |
| M_{K3[Fe(CN)6]}:M_{(FeSO4+citric acid)}=1:22 | 20 ppm | Yellowish-green spots, spot area/detection area≈70% | 95 - 100 s |
| Light yellow test paper | 30 ppm | Green spots, spot area/detection area≈85% | 95 - 100 s |
| | 40 ppm | Blue spots, spot area/detection area≈100% | 95 - 100 s |
| **Comparative Example 13** | 5 ppm | No visual change | - - |
| | 10 ppm | Light yellowish-green spots, spot area/detection area≈40% | 95 - 100 s |
| Distribution amount of K₃[Fe(CN)₆ on test paper: 0.4 mg/cm² | 20 ppm | Yellowish-green spots, spot area/detection area≈50% | 95 - 100 s |
| M_{K3[Fe(CN)6]}:M_{(FeSO4+citric acid)}=1:4 | 30 ppm | Green spots, spot area/detection area≈90% | 95 - 100 s |
| Orange test paper | 40 ppm | Blue spots, spot area/detection area≈100% | 95 - 100 s |
| **Comparative Example 14** | 5 ppm | No visual change | - - |
| | 10 ppm | No visual change | - - |
| Distribution amount of K₃[Fe(CN)₆ on test paper: 0.003 mg/cm² | 20 ppm | Very few light yellowish-green spots, spot area/detection area≈3% | 270 - 280 s |
| | 30 ppm | Light yellowish-green spots, spot area/detection area≈8% | 270 - 280 s |
| M_{K3[Fe(CN)6]}:M_{(FeSO4+citric acid)}=1:55 | 40 ppm | Yellowish-green spots, spot area/detection area≈10% | 270 - 280 s |
| White test paper | | | |

As shown in Table 9, the weight ratio of the K₃[Fe(CN)₆] to the powdery mixture of the FeSO₄ and the chelator has influence on detection sensitivity of the test paper. If the ratio of the K₃[Fe(CN)₆] is too high, the test paper is too dark in color, and the color change cannot be visually observed in the case of low free water content. If the ratio is two low, it also has the problems of detection failure due to low free water content and low detection sensitivity. Preferably, the ratio of the K₃[Fe(CN)₆] to the powdery mixture of the FeSO₄ and the chelator is 1:(11-22). The test paper can realize the color change process (when in contact with water) as: yellow (0 ppm), few light yellowish-green spots (5 ppm), light yellowish-green spots (10 ppm), yellowish-green spots (20 ppm), green spots (30 ppm) and blue spots (40 ppm), is green when the content of free water is 30 ppm (critical value) and is blue when the content of free water is 40 ppm, has color development at low free water content (lower than 20 ppm, such as 10 ppm), has obvious color change, fast and sensitive color development and easy judgment, can realize detection within 2 minutes, and has high detection efficiency. Most preferably, the ratio is 1:15, where the difference on the chromogenic reactions of different free water contents is obvious and easy to be visually observed.

### [Influence of Diameter of Fuel hole of Detection Device on Test Paper]

### Examples 1, 20-21 and comparative examples 15-16

The preparation process of the test paper is the same as in Example 1.

The aviation fuels with free water contents of 5 ppm, 10 ppm, 20 ppm, 30 ppm and 40 ppm are detected by the test paper, respectively. The detection is performed with the device shown in Fig.1. The diameters of the fuel holes of the employed detection devices in the examples and the comparative examples are listed in Table 10, the volume of the fuel sample being tested is 5 mL, three repeated experiments are set, and the average value or the average value standard deviation of the three repeated experiments is applied as result. The detection results are listed in Table 10.

**Table 10 Process Parameters and Detection Results in Examples 1, 20-21 and Comparative Examples 15-16**

| | **Detection result** | | **Detection time** |
|---|---|---|---|
| **Example 1** | 5 ppm | Few light yellowish-green spots, spot area/detection area≈5% | 65 - 70s |
| Diameter of fuel hole: 5 mm | 10 ppm | Light yellowish-green spots, spot area/detection area≈40% | 65 - 70s |
| | 20 ppm | Yellowish-green spots, spot area/detection area≈60% | 65 - 70s |
| | 30 ppm | Green spots, spot area/detection area≈80% | 65 - 70s |
| | 40 ppm | Blue spots, spot area/detection area≈100% | 65 - 70 s |
| **Example 20** | 5 ppm | Few light yellowish-green spots, spot area/detection area≈5% | 110 - 120 s |
| Diameter of fuel hole: 3 mm | 10 ppm | Light yellowish-green spots, spot area/detection area≈40% | 110 - 120 s |
| | 20 ppm | Yellowish-green spots, spot area/detection area≈60% | 110 - 120 s |
| | 30 ppm | Green spots, spot area/detection area≈80% | 110 - 120 s |
| | 40 ppm | Blue spots, spot area/detection area≈100% | 110 - 120 s |
| **Example 21** | 5 ppm | Few light yellowish-green spots, spot area/detection area≈5% | 60 - 65 s |
| Diameter of fuel hole: 7 mm | 10 ppm | Light yellowish-green spots, spot area/detection area≈50% | 60 - 65 s |
| | 20 ppm | Yellowish-green spots, spot area/detection area≈70% | 60 - 65 s |
| | 30 ppm | Green spots, spot area/detection area≈85% | 60 - 65 s |
| | 40 ppm | Blue spots, spot area/detection area≈100% | 95 - 100 s |
| **Comparative Example 15** | 5 ppm | Light yellowish-green spots, spot area/detection area≈10% | 240 - 250 s |
| Diameter of fuel hole: 2 mm | 10 ppm | Light yellowish-green spots, spot area/detection area≈60% | 240 - 250 s |
| | 20 ppm | Yellowish-green spots, spot area/detection area≈80% | 240 - 250 s |
| | 30 ppm | Green spots, spot area/detection area≈100% | 240 - 250 s |
| | 40 ppm | Blue spots, spot area/detection area≈100% | 240 - 250 s |
| **Comparative Example 16** | 5 ppm | Light yellowish-green spots, spot area/detection area≈10% | 60 - 65 s |
| | 10 ppm | Light yellowish-green spots, spot area/detection area≈50% | 60 - 65 s |
| Diameter of fuel hole: 8 mm | 20 ppm | Yellowish-green spots, spot area/detection area≈60% | 60 - 65 s |
| | 30 ppm | Green spots, spot area/detection area≈70% | 60 - 65 s |
| | 40 ppm | Blue spots, spot area/detection area≈80% | 60 - 65 s |

As shown in Table 10, the diameter of the fuel hole of the detection device has influence on detection sensitivity. If the diameter is too small, the detection time is too long, the accuracy of the detection result is low, and the difference on the chromogenic reactions in the case of high free water content is unobvious and not easy to be visually observed. Preferably, the diameter of the fuel hole is 3 - 7 mm. The test paper can realize the color change process (when in contact with water) as: yellow (0 ppm), few light yellowish-green spots (5 ppm), light yellowish-green spots (10 ppm), yellowish-green spots (20 ppm), green spots (30 ppm) and blue spots (40 ppm), is green when the content of free water is 30 ppm (critical value) and is blue when the content of free water is 40 ppm, has color development at low free water content (lower than 20 ppm, such as 10 ppm), has obvious color change, fast and sensitive color development and easy judgment, can realize detection within 2 minutes, and has high detection efficiency. Most preferably, the diameter is 5 mm, wherein the difference on the chromogenic reactions of different free water contents is obvious and easy to be visually observed.

### [Test Stability Detection]

The test papers prepared in Examples 1-21 are detected by the detection device shown in Fig.1. The aviation fuel with free water content of 40 ppm is detected by the test papers, respectively. The color residence times of the test papers are observed to obtain such a conclusion that green is stable at critical value (40 ppm) and is maintained for more than 24 hours under dry condition.

According to the tests hereinabove, the test paper for detecting free water in aviation fuels provided by the present invention has the advantages of simple detection method, high detection efficiency, low cost, long effective service life and high detection sensitivity. The detection sensitivity of the test paper can be regulated by regulating process parameters. The test paper can realize the color change process (when in contact with water) as yellow (0 ppm), few light yellowish-green spots (5 ppm), light yellowish-green spots (10 ppm), yellowish-green spots (20 ppm), green spots (30 ppm) and blue spots (40 ppm), and has obvious color change, fast and sensitive color development and easy judgment. At the critical value, the green is stable and is maintained for more than 24 hours under dry condition. The detection reliability is high.

Hereinabove mentioned are only the specific embodiments of the present invention, but the protection scope of the present invention is not limited thereto. In the technical scope disclosed by the present invention, any variations or substitutions readily conceivable by those skilled in the art should be covered within the protection scope of the present invention. Therefore, the protection scope of the present invention falls into the protection scope of the claims.

## Claims

1. A method for preparing a test paper to detect free water in aviation fuels, comprising the steps of:
soaking a filter paper in an aqueous solution of K₃[Fe(CN)₆];
drying the filter paper to obtain an intermediate test paper; and
coating a surface of the intermediate test paper with a powdery mixture of FeSO₄ and a chelator; wherein:
a weight ratio of the K₃[Fe(CN)₆] to the powdery mixture of the FeSO₄ and the chelator is 1 : (5 - 50).

2. The method in claim 1, wherein the filter paper is one of:
an oil filter paper;
a fast quantitative filter paper having a pore size of 80 - 120 µm; and
a fast qualitative filter paper having a pore size of 80 - 120 µm.

3. The method in claim 1, wherein a weight ratio of FeSO₄ to the chelator is 1 : (0.2% - 1%).

4. The method in one of claims 1-3, wherein the FeSO₄ has a particle size of 20 - 50 µm.

5. The method in one of claims 1-3, wherein the chelator includes one of ethylenediaminetetraacetic acid (EDTA), tartaric acid, tartrate salt, citric acid and citrate salt.

6. The method in claim 1, wherein a concentration of the aqueous solution of K₃[Fe(CN)₆] is 3 - 10 g/L.

7. The method in claim 1, wherein, in the drying step:
drying the filter paper at 40 - 80°C for 20 - 60 minutes to obtain the intermediate test paper.

8. The method in claim 1, further comprising:
preparing the powdery mixture of the FeSO₄ and the chelator, comprising the steps of:
mixing the FeSO₄ and the chelator; and
milling the powdery mixture of the FeSO₄ and the chelator until the powdery mixture has a particle size of 20 - 50 µm.

9. A device for detecting free water in aviation fuels, comprising:
the test paper (30) prepared with the method in one of claims 1-8;
a housing (10);
a core sleeve (20) being inserted into the housing (10) to form a sealing connection;
a cavity (12) formed between a front surface of the housing (10) and a front surface of the core sleeve (20);
a suction hole (21) which passes through the core sleeve (20); and
a fuel inlet (11) which passes through the front surface of the housing (10), wherein:
the cavity (12) communicates with the suction hole (21) and with the fuel inlet (11);
the test paper (30) is configured in the cavity (12); and
the aviation fuel being tested passes by the test paper (30) along a fuel channel formed by the fuel inlet (11), the cavity (12) and the suction hole (21).

10. The device in claim 9, wherein a diameter of the fuel inlet (11) is 3 - 7 mm.

## Patentansprüche

1. Verfahren zum Herstellen eines Testpapiers zum Nachweis von freiem Wasser in Flugkraftstoffen, umfassend die Schritte:
Eintauchen eines Filterpapiers in einer wässrigen Lösung von K₃[Fe(CN)₆];
Trocknen des Filterpapiers zur Erzeugung eines Zwischen-Testpapiers; und
Beschichten einer Oberfläche des Zwischen-Testpapiers mit einer pulverförmigen Mischung aus FeSO₄ und einem Chelatbildner, wobei:
das Gewichtsverhältnis von K₃[Fe(CN)₆] zu der pulverförmigen Mischung aus FeSO₄ und dem Chelatbildner 1: (5-50) beträgt.

2. Verfahren nach Anspruch 1, wobei das Filterpapier eines der folgenden ist:
ein Ölfilterpapier;
ein schnelles quantitatives Filterpapier mit einer Porengröße von 80 bis 120 µm, oder
ein schnelles qualitatives Filterpapier mit einer Porengröße von 80 bis 120 µm.

3. Verfahren nach Anspruch 1, wobei das Gewichtsverhältnis von FeSO₄ zu dem Chelatbildner 1: (0,2% - 1%) beträgt.

4. Verfahren nach einem der Ansprüche 1 bis 3, wobei das FeSO₄ eine Partikelgröße von 20 bis 50 µm aufweist.

5. Verfahren nach einem der Ansprüche 1 bis 3, wobei der Chelatbildner Ethylendiamintetraessigsäure (EDTA), Weinsäure, Tartarsalz, Zitronensäure oder ein Zitratsalz umfasst.

6. Verfahren nach Anspruch 1, wobei die Konzentration der wässrigen Lösung von K₃[Fe(CN)₆] is 3 - 10 g/l.beträgt.

7. Verfahren nach Anspruch 1, wobei im Trocknungsschritt:
das Filterpapier bei 40 bis 80 °C für 20 bis 60 Minuten getrocknet wird.

8. Verfahren nach Anspruch 1, ferner umfassend:
Herstellen der pulverförmigen Mischung aus FeSO₄ und dem Chelatbildner, umfassend die Schritte:
Mischen des FeSO₄ und des Chelatbildners; und
Mahlen der pulverförmigen Mischung aus FeSO₄ und dem Chelatbildner, bis die pulverförmige Mischung eine Partikelgröße von 20 bis 50 µm aufweist.

9. Vorrichtung zum Nachweis von freiem Wasser in Flugkraftstoffen, umfassend:
das mit dem Verfahren nach einem der Ansprüche 1 bis 8 hergestellte Testpapier (30);
ein Gehäuse (10);
eine Kernhülse (20), die in das Gehäuse (10) eingefügt ist, um eine Dichtverbindung zu bilden;
einen Hohlraum (12), der zwischen einer Vorderseite des Gehäuses (10) und einer Vorderseite der Kernhülse (20) gebildet ist;
ein Saugloch (21), das die Kernhülse (20) durchläuft; und
einen Kraftstoffeinlass (11), der die Vorderseite des Gehäuses (10) durchläuft, wobei:
der Hohlraum (12) mit dem Saugloch (21) und dem Kraftstoffeinlass (11) in Verbindung steht;
das Testpapier (30) im Hohlraum (12) angeordnet ist, und
der zu prüfende Flugkraftstoff entlang eines durch den Kraftstoffeinlass (11), den Hohlraum (12) und das Saugloch (21) gebildeten Kraftstoffkanals an dem Testpapier (30) vorbeigeführt wird.

10. Vorrichtung nach Anspruch 9, wobei der Durchmesser des Kraftstoffeinlasses (11) 3 bis 7 mm beträgt.

## Revendications

1. Procédé de préparation d'un papier test pour détecter l'eau libre dans les carburants d'aviation, comprenant les étapes de :
trempe d'un papier filtre dans une solution aqueuse de K₃[Fe(CN)₆] ;
séchage du papier filtre pour obtenir un papier test intermédiaire ; et
revêtement d'une surface du papier test intermédiaire avec un mélange pulvérulent de FeSO₄ et d'un agent chélatant ; dans lequel :
un rapport pondéral du K₃[Fe(CN)₆] par rapport au mélange pulvérulent du FeSO₄ et de l'agent chélatant est de 1: (5 - 50).

2. Procédé selon la revendication 1, dans lequel le papier filtre est l'un parmi :
un papier filtre à huile ;
un papier filtre quantitatif rapide présentant une taille de pores de 80-120 µm ; et
un papier filtre qualitatif rapide présentant une taille de pores de 80 - 120 µm.

3. Procédé selon la revendication 1, dans lequel un rapport pondéral du FeSO₄ par rapport à l'agent chélatant est de 1: (0.2% - 1%).

4. Procédé selon l'une des revendications 1-3, dans lequel le FeSO₄ présente une granulométrie de 20 - 50 µm.

5. Procédé selon l'une des revendications 1-3, dans lequel l'agent chélatant comporte l'un parmi l'acide éthylènediaminetétraacétique (EDTA), l'acide tartrique, un sel tartrate, l'acide citrique et un sel citrate.

6. Procédé selon la revendication 1, dans lequel une concentration de la solution aqueuse de K₃[Fe(CN)₆] est de 3 - 10 g/L.

7. Procédé selon la revendication 1, dans lequel, à l'étape de séchage :
séchage du papier filtre à 40 - 80°C pendant 20 - 60 minutes pour obtenir le papier test intermédiaire.

8. Procédé selon la revendication 1, comprenant en outre :
la préparation du mélange pulvérulent du FeSO₄ et de l'agent chélatant, comprenant les étapes de :
mélange du FeSO₄ et de l'agent chélatant ; et
broyage du mélange pulvérulent du FeSO₄ et de l'agent chélatant jusqu'à ce que le mélange pulvérulent ait une granulométrie de 20 - 50 µm.

9. Dispositif de détection d'eau libre dans les carburants d'aviation, comprenant :
le papier test (30) préparé selon le procédé de l'une des revendications 1-8 ;
un boîtier (10) ;
un manchon central (20) étant inséré dans le boîtier (10) pour former une connexion d'étanchéité étanche ;
une cavité (12) formée entre une surface avant du boîtier (10) et une surface avant du manchon central (20) ;
un trou d'aspiration (21) qui passe à travers le manchon central (20) ; et
une entrée de carburant (11) qui passe à travers la surface avant du boîtier (10), dans lequel :
la cavité (12) est en communication avec le trou d'aspiration (21) et l'entrée de carburant (11) ;
le papier test (30) est configuré dans la cavité (12) ; et
le carburant d'aviation testé passe à travers le papier test (30) le long d'un canal de carburant formé par l'entrée de carburant (11), la cavité (12) et le trou d'aspiration (21).

10. Dispositif selon la revendication 9, dans lequel un diamètre de l'entrée de carburant (11) est de 3 - 7 mm.
